# EUROPÄISCHE PATENTSCHRIFT

(11) **EP 1 059 920 B1**
(45) Veröffentlichungstag und Bekanntmachung des Hinweises auf die Patenterteilung: **03.07.2002**
(21) Anmeldenummer: 98965783.8
(22) Anmeldetag: 08.12.1998
(51) Int. Cl.: A61K 31/19, A61K 9/50

(54) **VERWENDUNG VON ALKYLHYDROGENFUMARATEN ZUR BEHANDLUNG VON PSORIASIS, PSORIATISCHER ARTHRITIS, NEURODERMITIS UND ENTERITIS REGIONALIS CROHN**
USE OF ALKYL HYDROGEN FUMARATES FOR TREATING PSORIASIS, PSORIATIC ARTHRITIS, NEURODERMATITIS AND REGIONAL ENTERITIS
UTILISATION DE FUMARATES D'HYDROGENE D'ALKYLE POUR LE TRAITEMENT DU PSORIASIS, DE LA POLYARTHRITE PSORIASIQUE, DE LA NEURODERMITE ET DE L'ENTERITE REGIONALE

(30) Priorität: 31.03.1998 DE 19814358
(43) Veröffentlichungstag der Anmeldung: 20.12.2000
(73) Patentinhaber: Fumapharm AG, 5630 Muri (CH)
(72) Erfinder: JOSHI, Rajendra, K., CH-8048 Zürich (CH); STREBEL, Hans-Peter, CH-5630 Muri (CH)
(74) Vertreter: Schwabe - Sandmair - Marx
(86) Internationale Anmeldenummer: EP9807956
(87) Internationale Veröffentlichungsnummer: WO9949858

(56) Entgegenhaltungen:
- EP-A- 0 312 697
- DE-A- 3 834 794
- NIBBERING P.H. ET AL: "Effects of monomethylfumarate on human granulocytes." JOURNAL OF INVESTIGATIVE DERMATOLOGY, (1993) 101/1 (37-42). ISSN: 0022-202X CODEN: JIDEAE, XP002107171 United States
- ALTMEYER P. ET AL: "[Systemic therapy of psoriasis ]. SYSTEMISCHE THERAPIE DER PSORIASIS." THERAPIE UND ERFOLG DERMATOLOGIE, (1997) 27/6 (380-384). ISSN: 0939-0448 CODEN: TEDEFX, XP002107172 Germany
- NIBBERING, P.H. ET AL: "Intracellular signalling by binding sites for the antipsoriatic agent monomethylfumarate on human granulocytes" BR. J. DERMATOL. (1997), 137(1), 65-75 CODEN: BJDEAZ;ISSN: 0007-0963, XP002107173
- SEBOEK, BELA ET AL: "Antiproliferative and cytotoxic profiles of antipsoriatic fumaric acid derivatives in keratinocyte cultures" EUR. J. PHARMACOL., ENVIRON. TOXICOL. PHARMACOL. SECT. (1994), 270(1), 79-87 CODEN: EPEPEG, XP002107174

## Beschreibung

Die Erfindung betrifft die Verwendung der freien Säureform bestimmter Fumarsäuremonoalkylester (Alkylhydrogenfumarate) allein oder in Verbindung mit einem Dialkylfumarat zur Herstellung einer pharmazeutischen Zubereitung in Form von Mikrotabletten zur Behandlung der Psoriasis, psoriatischen Arthritis, Neurodermitis und Enteritis regionalis Crohn.

Pharmazeutische Zubereitungen, die nach Verabreichung bei ihrem biologischen Abbau in den Zitronensäurezyklus einmünden oder diesem angehören, gewinnen meist in hoher Dosierung immer mehr an therapeutischem Wert, da man mit ihrer Hilfe kryptogenetisch bedingte Krankheiten zu lindern oder zu heilen vermag.

So hemmt Fumarsäure das Wachstum des Ehrlich-Ascites-Tumors bei Mäusen, vermindert die toxischen Effekte von Mitomycin C und Aflatoxin (K. Kuroda, M. Akao, Biochem. Pharmacol. 29, 2839-2844 (1980) / Gann. 72, 777-782 (1981) / Cancer Res. 36, 1900-1903 (1976)) und besitzt eine antipsoriatische sowie antimikrobielle Wirkung (C. N. Huhtsnen. J. Food Sci. 48, 1574 (1983) / M. N. Islam, US-A-4 346 118 / C. A. 97, 161317b (1982)).

Hohe Verabreichungsdosen von bisher hierfür bekannten Fumarsäurederivaten wie Dihydroxyfumarsäure. Fumaramid und Fumaronitril besitzen bei parenteraler, dermaler, insbesondere aber peroraler Verabreichung eine derart unzumutbare Nebenwirkungsrate und hohe Toxizität (P. Holland. R. G. White, Brit. J. Dermatol. 85, 259-263 (1971) / M. Hagedorn, K. W. Kalkoff. G. Kiefer, D. Baron. J. Hug, J. Petres. Arch. Derm. Res. 254, 67-73 (1975)), daß bisher meist von einer solchen Therapie abgesehen werden mußte.

In der EP-A-0 188 749 sind bereits Fumarsäurederivate (Salze) und diese enthaltende pharmazeutische Zubereitungen zur Behandlung der Psoriasis beschrieben.

Aus der DE-A-25 30 372 sind pharmazeutische Zubereitungen zur Behandlung der Psoriasis bekannt, die eine Mischung aus Fumarsäure und weiteren Fumarsäurederivaten enthalten. Ein Anteil an Fumarsäure ist obligatorisch.

Die DE-A-26 21 214 beschreibt Arzneimittel zur Behandlung der Psoriasis, die Fumarsäuremonoethylester und dessen Mineralsalze als Wirkstoff enthalten. Weiterhin beschreibt die Europäische Patentanmeldung 0 312 697 die Verwendung verschiedener Fumarsäuremonoalkylestersalze zur Therapie von Psoriasis, der psoriatischen Arthritis, der Neurodermitis sowie des der Enteritis regionalis Crohn.

Aus der Publikation "Hautarzt (1987) 279-285" ist die Verwendung von Fumarsäuremonoethylestersalzen (Ca, Zn, Mg) und Fumarsäuredimethylester für die Psoriasisbehandlung bekannt.

Da in einer psoriatischen Epidermis die Aktivität der Phospholipase A₂ verändert ist. liegt eine mögliche Erklärung des Wirkungsmechanismus der erfindungsgemäßen Zubereitungen darin, daß dieses Enzym durch Fumarsäure stimuliert wird.

Es wurde nunmehr überraschend gefunden, daß eine Behandlung der Psoriasis auch mit Alkylhydrogenfumarate selbst ohne Salzbildung durch eine pharmazeutische Zubereitung erzielt werden kann, die die freie Säureform eines oder mehrerer C₁₋₅-Alkylhydrogenfumarate und ggf. übliche pharmazeutisch verträgliche Hilfs- und Trägerstoffe enthält und in Form von Mikrotabletten oder Mikropallets vorliegt. Gegebenenfalls können diese Zubereitungen außerdem ein oder mehrere Dialkylfumarate enthalten.

Die erfindungsgemäßen Zubereitungen in Form von Mikrotabletten oder Mikropellets gestatten die Verabreichung der freien Saure anstelle ihrer Salze ohne Auftreten der bekannten Nebenwirkungen, insbesondere ohne Ausbildung von Ulcera. Dies beruht vermutlich darauf, daß die Mikrotabletten oder Mikropellets eine gleichmäßige Verteilung im Magen gestatten, wodurch lokale, reizende Konzentration des Monoalkylhydrogenfumarats als freie Säure vermieden werden.

Zur peroralen Verabreichung sind besonders Zubereitungen geeignet, die die freie Säure des Alkylhydrogenfumarats in einer Menge von 20 bis 300 mg enthalten, wobei das Gesamtgewicht der Wirkstoffe 100 bis 300 mg beträgt.

Für den systemischen Einstieg in die Behandlung bzw. den Ausstieg ist eine niedrige Dosierung vorteilhaft, die beispielsweise 100 bis 120 mg Wirkstoff enthält, beispielsweise 30,0 mg bis 35,0 mg Dimethylfumarat und 70 bis 90 mg Methylhydrogenfumarat.

Für die therapeutische Dosierung nach der Einstiegsphase kann beispielsweise eine Dosierung von 190 - 210 mg Wirkstoff z.B. in Form von 120,0 mg Dimethylfumarat und 90,0 mg Monoethylfumarat zur Anwendung kommen.

Die erfindungsgemäßen Zubereitungen werden peroral in Form von Mikrotabletten oder in Kapseln befindlichen Mikrotabletten oder Mikropellets verabreicht. wobei sich die festen Einzeldosisarzneiformen im Magen innerhalb weniger Minuten lösen und das aktive Prinzip gleichmäßig verteilt aus der Arzneiform freisetzen. Zum systematischen Einstieg bzw. Ausstieg ist eine niedrige Dosierung erforderlich, für die therapeutische Dosierung nach der Einstiegsphase eine höhere Dosierung.

Die Mikrotabletten der vorliegenden Erfindung werden gemäß der im Stand der Technik bekannten Verfahren hergestellt wie dem Granulieren. Sieben, Extrusion/Spheronisation usw.. Sie können zusätzlich zum Wirkstoff übliche Hilfs- und Trägerstoffe wie Stärke. Lactose. PVP und dergleichen enthalten. Die Mikrotabletten bzw. -pellets haben vorzugsweise eine Größe von 300 - 2000 µm. stärker bevorzugt 500 - 1500 µm und noch stärker bevorzugt von 1000 µm.

Zur vereinfachten Verabreichung der Einzeldosis können die Mikrotabletten bzw. -pellets in Kapseln z,B. Gelatinekapseln gefüllt werden. Wahlweise können die Mikrotabletten bzw. -pellets mit einem magensaftresistenten Überzug versehen sein. Dieser kann gemäß üblicher Verfahren auf die Tabletten aufgebracht werden z.B. mittels Aufleeren oder Aufsprühen in einem Wirbelschichtapparat oder als Filmcoat.

### Beispiel

Herstellung von Mikropellets in Kapseln enthaltend 50,0 mg Methylhydrogenfumarat, entsprechend insgesamt 44,6 mg Fumarsäure

5.000 kg Methylhydrogenfumarat werden zerkleinert und mittels eines Siebes 400 unter entsprechenden Vorsichtsmaßnahmen (Atemmaske, Handschuhe, Schutzanzug etc.) homogenisiert. Daneben werden 2 l einer 20% (m/V) Polyvinylpyrrolidon (Kollidon K30)-Lösung in Ethanol vorbereitet. 7,250 kg Nonpareilles Pellets werden in einem Dragierkessel vorgelegt und mit einem Teil der Kollidon K-30-Lösung besprüht bis diese leicht feucht werden. Das Wirkstoffgemisch wird danach portionsweise zugegeben bis zum Auftrocknen der Pellets. Diese Vorgehensweise der Befeuchtung/Auftrocknung wird bis zur endgültigen Zugabe des Wirkstoffgemisches weitergeführt. Die Pellets werden letztlich bis zum vollständigen Austrocknen bewegt. Die Pellets werden danach in Hartgelatinekapseln abgefüllt (126,5 mg Pellets/Kapsel).

Es wurde festgestellt, daß die erfindungsgemäßen Zubereitungen eine den bekannte Fumarsäurederivate als Salze enthaltenden Präparaten vergleichbare Wirkung gegen die verschiedensten klinischen Erscheinungsformen der Psoriasis, der psoriatischen Arthritis, der Neurodermitis sowie der Enteritis regionalis Crohn (Morbus Crohn) autweisen. jedoch frei von den für die Verabreichung der freien Säure bekannten Nebenwirkungen sind.

### Untersuchung der akuten Toxizität

Die akute Toxizität wurde vor der klinischen Prüfung an Ratten mit Methylhydrogenfumarat peroral untersucht. Die Resultate zeigten eine sehr geringe Toxizität der eingesetzten Fumarsäuren (s. Tabelle 2).

**Tabelle 1:**

| Akute Toxizitätsstudie (oral) an der Ratte | | |
|---|---|---|
| | Geschlecht | Methylhydrogenfumarat |
| LD₅₀ | männlich | 2606.8 |
| | weiblich | 1777.8 |
| "Lowest Lethal Dose" in mg/kg | männlich | 2150 |
| | weiblich | 1470 |

### Pharmazeutische Äquivalenz

Vergleich der pharmakokinetischen Daten von Fumaderm forte® (Bsp. 4 aus EP-Patent 0 312 697 B1) und Monomethylfumarat bzw. Monoethylfumarat als Calciumsalz.

**Tabelle 2:**

| Äquivalenz | | | |
|---|---|---|---|
| Verabreichte Substanz | Spezies | Dosis [mg/kg K.G] | Methylhydrogenfumaratspiegel (Cₘₐₓ) [µg/ml] |
| Fumaderm forte | Ratte | 30 | 8.99 |
| Methylhydrogenfumarat | Ratte | 100 | m: 69.9 |
| | | | f: 84.8 |
| Methylhydrogenfumarat Calciumsalz | Ratte | 100 | m: 51.3 |
| | | | f: 107.0 |
| Fumaderm forte: Die Mischung enthält 120 mg Dimethylfumarat, 87 mg Monoethylfumarat Calciumsalz, 5 mg Monoethylfumarat Magnesiumsalz, 3 mg Monoethylfumarat Zinksalz | | | |

## Patentansprüche

1. Verwendung einer oder mehrerer Alkylhydrogenfumarate der allgemeinen Formel 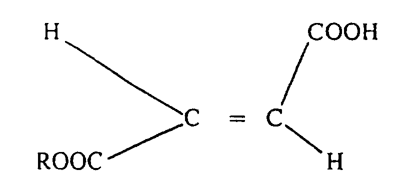 in der R ein C₁₋₅-Alkyl ist. gegebenenfalls im Gemisch mit Dialkylfumarat der Formel 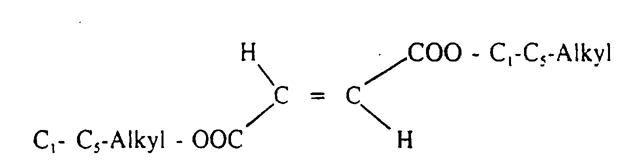 und ggf. üblicher pharmazeutischer Hilfs- und Trägerstoffe zur Herstellung einer pharmazeutischen Zubereitung in Form von Mikrotabletten oder Mikropellets zur Behandlung der Psoriasis, der psoriatischen Arthritis, Neurodermitis und Enteritis regionalis Crohn.

2. Verwendung nach Anspruch 1, **dadurch gekennzeichnet, daß** es sich um das Methylhydrogenfumarat handelt.

3. Verwendung nach Anspruch 1, **dadurch gekennzeichnet, daß** es sich um das Methylhydrogenfumarat im Gemisch mit Dimethylfumarat handelt.

4. Verwendung nach einem der Ansprüche 1 bis 3 zur Herstellung einer pharmazeutischen Zubereitung zur peroralen Verabreichung in Form von Mikrotabletten oder Mikropellets, **dadurch gekennzeichnet, daß** das Gesamtgewicht der Wirkstoffe 20 bis 300 mg beträgt.

5. Verwendung nach einem der Ansprüche 1 bis 4, **dadurch gekennzeichnet, dass** die Zubereitung 10 bis 290 Gewichtsteile Methylhydrogenfumarat und 290 bis 10 Gewichtsteile Dimethylfumarat enthält.

6. Verwendung nach einem der vorhergehenden Ansprüche zur Herstellung einer pharmazeutischen Zubereitung zur peroralen Verabreichung, **dadurch gekennzeichnet, daß** die Mikrotabletten oder Mikropellets mit einem magensaftresistenten Überzug versehen sind.

7. Verwendung gemäß einem der vorstehenden Ansprüche, **dadurch gekennzeichnet. daß** die Mikrotabletten oder Mikropellets eine Größe von 300 bis 2000 µm haben.

## Claims

1. The use of one or several alkyl hydrogen fumarates of the general formula 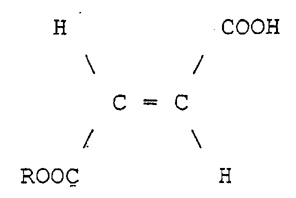 wherein R is a C₁₋₅ alkyl, optionally in admixture with dialkyl fumarate of the formula 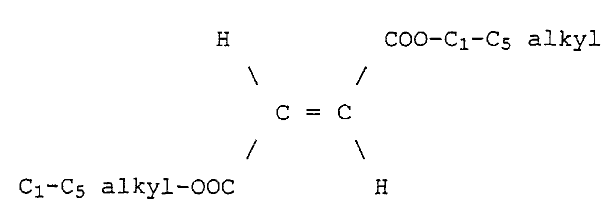 and optionally customary pharmaceutical excipients and carriers for preparing a pharmaceutical composition in the form of micro-tablets or micro-pellets for treating psoriasis, psoriatic arthritis, neurodermatitis and enteritis regionalis Crohn.

2. The use according to claim 1, **characterised in that** methyl hydrogen fumarate is used.

3. The use according to claim 1, **characterised in that** methyl hydrogen fumarate mixed with dimethyl fumarate is used.

4. The use according to one of the claims 1 to 3 for preparing a pharmaceutical composition for oral adminstration in the form of micro-tablets or micro-pellets in capsules, **characterized in that** the oral weight of the active ingredients is 20 to 300 mg.

5. The use according to one of claims 1 to 4, **characterised in that** the preparation contains 10 to 290 parts by weight of methyl hydrogen fumarate and 290 to 10 parts by weight of dimethyl fumarate.

6. The use according to one of the preceding claims for preparing a pharmaceutical composition for oral administration, **characterised in that** the micro-pellets are provided with an enteric coating.

7. The use according to one of the preceding claims, **characterised in that** the micro-tablets or micro-pellets have a size of 300 to 2,000 µm.

## Revendications

1. Utilisation d'un ou de plusieurs fumarates de monoalkyle de formule générale 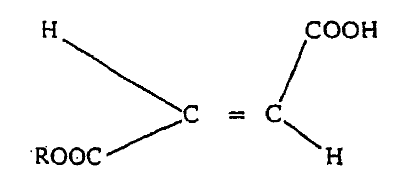 dans laquelle R est un alkyle en C₁₋₅, éventuellement en mélange avec du fumarate de dialkyle de formule 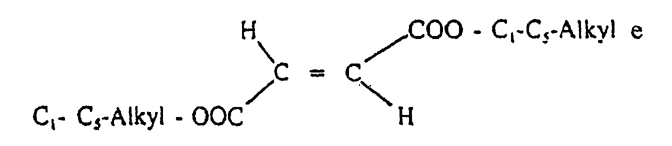 et éventuellement des adjuvants et supports pharmaceutiques usuels pour la fabrication d'une préparation pharmaceutique sous la forme de microcomprimés ou de micropastilles pour le traitement du psoriasis, de l'arthrite psoriasique, de la neurodermite et de l'entérite régionale de Crohn.

2. Utilisation selon la revendication 1, **caractérisée en ce qu'**il s'agit de l'hémifumarate de méthyle.

3. Utilisation selon la revendication 1, **caractérisée en ce qu'**il s'agit de l'hémifumarate de méthyle en mélange avec le fumarate de diméthyle.

4. Utilisation selon l'une des revendications 1 à 3 pour la fabrication d'une préparation pharmaceutique pour administration perorale sous la forme de microcomprimés ou de micropastilles en capsules, **caractérisée en ce que** la masse totale de la substance active est de 20 à 300 mg.

5. Utilisation selon l'une des revendications 1 à 4, **caractérisée en ce que** la préparation contient de 10 à 290 parties en poids d'hémifumarate de méthyle et de 290 à 10 parties en poids de fumarate de diméthyle.

6. Utilisation selon l'une des revendications précédentes pour la fabrication d'une préparation pharmaceutique pour administration perorale, **caractérisée en ce que** les microcomprimés ou les micropastilles sont munies d'un revêtement résistant au suc gastrique.

7. Utilisation selon l'une des revendications précédentes, **caractérisée en ce que** les microcomprimés ou les micropastilles ont une taille de 300 à 2000 µm.
